# FASCICULE DE BREVET EUROPEEN

(11) **EP 0 867 445 B1**
(45) Date de publication et mention de la délivrance du brevet: **09.10.2002**
(21) Numéro de dépôt: 98480018.5
(22) Date de dépôt: 23.03.1998
(51) Int. Cl.: C07F 7/18, A61K 7/48, A61K 7/06, C07F 9/40, C07F 9/09

(54) **Complexes à base silicium biologiquement actif sous forme solide**
Auf biologisches aktives Silicium basierte Komplexe in fester Form
Complexes based on biologically silicon in solid form.

(30) Priorité: 24.03.1997 FR 9703792
(43) Date de publication de la demande: 30.09.1998
(73) Titulaire: EXSYMOL S.A.M., MC-98000 Monte Carlo (MC)
(72) Inventeur: Seguin, Marie-Christine, 98000 Monaco (MC); Gueyne, Jean, 98000 Monaco (MC)
(74) Mandataire: Bonneau, Gérard

(56) Documents cités:
- EP-A- 0 289 366
- FR-A- 2 158 068
- FR-A- 2 454 803
- FR-A- 2 561 915
- FR-A- 2 610 522
- PATENT ABSTRACTS OF JAPAN vol. 12, no. 209 (C-504) [3056] , 15 juin 1988 & JP 63 008390 A (LION CORP.), 14 janvier 1988,
- CHEMICAL ABSTRACTS, vol. 106, no. 9, 2 mars 1987 Columbus, Ohio, US; abstract no. 067477, NODA S ET AL: "Organosilanol compositions" XP002049173 & JP 61 129 185 A (LION CORP.;JAPAN) 17 juin 1986
- CHEMICAL ABSTRACTS, vol. 106, no. 9, 2 mars 1987 Columbus, Ohio, US; abstract no. 067478, NODA S ET AL: "Organosilanol compositions" XP002049174 & JP 61 129 186 A (LION CORP.;JAPAN) 17 juin 1986
- FRANCO A: "Silanol procosmetics" COSMET. NEWS (COSNDG);88; VOL.60,; PP.163-8, SOC. EXYMOL;MONACO; MONACO (MC), XP002049172

## Description

La présente invention a pour objet des complexes à base de composés organosiliciés, qui sont sous forme solide, et une composition pharmaceutique ou cosmétique les contenant. Cet objectif de complexe est atteint en établissant des liaisons faibles ou fortes entre lesdits composés organosiliciés et un stabilisant.

Les composés à base de silicium biologiquement actif sont des composés organo-siliciés et plus particulièrement des silanols présentant plusieurs groupements Si-OH. A l'instar des brevets FR-A-2610522 et EP-A-0289366, les silanols sont décrits dans l'état de la technique comme constituant une forme de silicium assimilable par l'organisme, à condition toutefois de posséder la propriété d'exister en solution aqueuse sous forme d'oligomères solubles de faible poids moléculaire. D'autres brevets font état également de composés silanolés présentés et stabilisés en solution, tels les composés figurant dans des compositions ou produits à usage cosmétique (brevets JP 61129185, JP 61129186 et FR-A-2561915) ou thérapeutique (brevet FR-A-2158068).

Jusqu'à présent, les composés organo-siliciés biologiquement actifs n'ont donc été disponibles que sous forme de solutions diluées, car ils polymérisent lorsqu'ils sont trop concentrés, et à l'exemple du brevet JP 63008390 toutes les tentatives d'obtention de ces composés par élimination d'eau ont jusqu'à ce jour invariablement conduit à leur polymérisation, la conséquence d'un tel phénomène étant la perte de leurs propriétés biologiques.
Le fait que les composés organosiliciés de l'art antérieur n'existent que dans des solutions diluées limite leur utilisation et notamment rend impossible leur incorporation dans des compositions cosmétiques ou thérapeutiques se présentant sous forme non aqueuse ou sous forme de comprimés ou cachets destinés à être administrés par voie orale.

Ainsi, pour notamment remédier à ces inconvénients, un des buts principaux de l'invention est l'obtention d'un complexe à base d'un composé organosilicié biologiquement actif qui se présente sous forme solide.

Le composé constitutif du complexe selon l'invention répond à la formule générale (I) suivante :

R₄ - Si (OR₁) (OR₂) (R₃) (I)

dans laquelle
R₁ et R₂ chacun indépendamment représentent un atome d'hydrogène ou un groupement alkyle,
R₃ représente un atome d'hydrogène, un groupement hydroxyle, un groupement amine, un groupement alkyle, un groupement alcoxy ou un groupement carboxylate,
et au moins un dès groupements OR₁, OR₂ ou R₃ représente un groupement hydroxyle,
R₄ représente un groupement alkyle substitué ou non par un groupement fonctionnel tel que notamment un groupement alkylphosphate ou alkylphosphonate, ou tout autre groupement tel que la liaison R₄-Si n'est pas hydrolysable, ledit complexe étant caractérisé en ce que :
- il est sous forme solide,
- il résulte de la formation de liaisons faibles ou fortes entre le composé organosilicié et au moins un stabilisant.

De préférence, le composé constitutif du complexe selon l'invention est un composé de formule (I) dans laquelle R₄ représente un groupement diéthylphosphatoéthyle et/ou les groupements OR₁, OR₂ et R₃ représentent chacun un groupement hydroxyle.

Suivant un mode avantageux de réalisation de l'invention, le stabilisant est un acide carboxylique, un acide aminé ou un dérivé d'acide aminé, un peptide ou une protéine, un alcool ou un polyol, un polysaccharide, et/ou leurs sels.

De préférence, l'acide carboxylique et/ou son sel est l'acide aspartique, l'acide glutamique, l'acide lactique, l'acide salicylique, l'acide théophylline acétique, l'acide pyrrolidinone carboxylique et/ou leurs sels.

De préférence, l'acide aminé et/ou son sel est l'arginine, la sérine, la thréonine, l'hydroxyproline, l'acétylméthionine, l'acétyl tyrosine et/ou leurs sels.

De préférence, le, peptide est un polypeptide dérivé de protéines d'élastine, de spiruline, de collagène, ou de protéines végétales, telles que notamment les protéines d'avoine ou de blé.

Avantageusement, ce polypeptide est un hydrolysat de protéines d'élastine, de spiruline, de collagène, ou de protéines végétales, telles que notamment les protéines d'avoine ou de blé.

De préférence, la protéine est une protéine d'élastine, de spiruline, de collagène, ou une protéine végétale, telle que notamment une protéine d'avoine ou de blé.

Avantageusement, le polyol est du lactose.

De préférence, le polysaccharide est un glycosaminoglycanne, un mucopolysaccharide tel que notamment l'acide hyaluronique, la pectine ou l'acide alginique.

Suivant un autre mode de réalisation de l'invention, le dispersant est un polyamide, un carbohydrate, un polysaccharide, un polyoxyéthylène tel qu'un polyéthylène glycol ou un corps gras non soluble dans l'eau ou faiblement soluble dans l'eau tel que notamment un triglycéride.

Un autre but de l'invention est de proposer une composition pharmaceutique ou cosmétique comprenant un tel complexe, en association avec tout excipient approprié, et qui soit administrable par voie orale, de manière à être utilisable en médication ambulatoire ne nécessitant pas la réalisation d'un acte médical préalable.

L'invention se comprendra mieux à la lecture de la description détaillée qui suit ; celle-ci a pour but d'illuster et d'expliciter, sans la limiter, l'invention.

Le composé constitutif du complexe selon l'invention répond à la formule générale (I) dans laquelle R₁ et R₂ chacun indépendamment représentent un atome d'hydrogène ou un groupement alkyle, de préférence un groupement C₁₋₄ alkyle, R₃ représente un atome d'hydrogène, un groupement hydroxyle, un groupement amine, un groupement alkyle, de préférence C₁₋₄ alkyle, un groupement alcoxy, de préférence C₁₋₄ alcoxy ou un groupement carboxylate, de préférence lié au silicium par un atome d'oxygène, et au moins un des groupements OR₁, OR₂ ou R₃ représente un groupement hydroxyle, R₄ représente un groupement alkyle substitué ou non tel que notamment un groupement alkylphosphate, de préférence C₁₋₄ alkylphosphate ou alkylphosphonate de préférence, C₁₋₄ alkylphosphonate, ou tout autre groupement tel que la liaison R₄-Si n'est pas hydrolysable, ledit complexe ayant une caractéristique principale, qui est de se présenter sous forme solide. Ce complexe résulte obligatoirement de l'association avec au moins un stabilisant.

Les complexes préférés de l'invention sont ceux à base de composés organosiliciés de formule (I) dans lesquels R₄ représente un groupement diéthylphosphatoéthyle.
Parmi ces composés, il est à distinguer le diéthylphosphatoéthylsilanetriol.

Une caractéristique du composé organosilicié constituant le complexe selon l'invention est que une partie des substituants de l'atome de silicium peuvent, au contact d'une molécule d'eau, s'hydrolyser spontanément pour former une liaison Si-OH supplémentaire, ce qui contribue à renforcer l'activité *in vivo* desdits composés.

Le stabilisant est un composé polaire pouvant former des liaisons faibles (liaisons hydrogène) ou fortes (liaisons covalentes) avec le composé organo-silicié. Le stabilisant s'oppose à la formation d'une liaison siloxane Si-O-Si conduisant à des dérivés polysiloxanes peu ou pas solubles dans l'eau. Il va contribuer à éviter la polymérisation du composé organo-silicié.

Par exemple, le stabilisant peut être un acide carboxylique ou son sel, tel que notamment l'acide aspartique, l'acide glutamique, l'acide lactique, l'acide salicylique, l'acide théophylline acétique, l'acide pyrrolidinone carboxylique et/ou leurs sels.

Le stabilisant peut également être un acide aminé ou un dérivé d'acide aminé et/ou leurs sels. Parmi les acides aminés, la sérine, la thréonine, l'hydroxyproline, l'acétylméthionine, l'acétyl tyrosine et/ou leurs sels sont préférés.

L'hydroxyproline est particulièrement intéressante en outre pour sa propriété régénérante du tissu conjonctif. A l'état naturel, elle est présente en quantité élevée dans le collagène.

De même, l'acétyl tyrosine est en outre intéressante pour son rôle dans la mélanogénèse.

Le stabilisant peut donc aussi être un peptide ou une protéine. Parmi les peptides, les polypeptides dérivés de protéines d'élastine, de spiruline, de collagène, ainsi que les polypeptides dérivés de protéines végétales tels que notamment de protéines d'avoine ou de blé sont préférés. Avantageusement, on choisit parmi ces polypeptides ceux qui résultent de la lyse ou de l'hydrolyse des protéines. Notamment, on obtient de bons résultats en termes de stabilisation avec des hydrolysats de protéine de blé.

De même, le stabilisant peut être une protéine et dans ce cas, de la même façon, les protéines d'élastine, de spiruline, de collagène, ainsi que les protéines végétales tels que notamment de protéines d'avoine ou de blé sont préférés.

Le stabilisant peut encore être un alcool ou un polyol tel que le lactose.

Le stabilisant peut également être un polysaccharide. Parmi les polysaccharides, les glucosaminoglycanes, les mucopolysaccharides tel que notamment l'acide hyaluronique, la pectine et l'acide alginique sont préférés.

Un bon stabilisant, par exemple, est l'acide hyaluronique cité plus haut, qui est en fait le mucopolysaccharide acide résultant de la réaction entre le N-acétyl-glucosamine avec l'acide glucuronique.

Tous ces composés sont choisis comme stabilisants parce qu'ils ont les propriétés ci-dessus énoncées, et aussi parce qu'ils sont couramment employés en cosmétique.

Suivant un mode de réalisation particulier de l'invention, le complexe peut également résulter de l'association avec un dispersant.
Le dispersant a pour objectif de diluer le composé organosilicié et de s'opposer à sa polycondensation.
Le dispersant se présente donc sous la forme d'une matrice plus ou moins inerte visant à diluer dans la poudre les molécules de composé organo-silicié. Bien que de formule chimique parfois assez proche de celle du stabilisant, le dispersant ne contribue pas à la stabilisation directe du composé organo-silicié : il y contribue principalement par l'effet de dilution.

L'addition d'un dispersant peut également être motivée par des considérations liées à la formulation des compositions selon l'invention.

Suivant un autre mode de réalisation de l'invention, le dispersant est un polyamide, un carbohydrate, un polysaccharide, un polyoxyéthylène tel qu'un polyéthylène glycol ou un corps gras non soluble dans l'eau ou faiblement soluble dans l'eau; tel que notamment un triglycéride.
Avantageusement, le carbohydrate est du sorbitol.

De préférence, le polysaccharide est de la cellulose.

Le dispersant et le stabilisant peuvent avoir des propriétés physico-chimiques communes.

En présence d'eau, le composé organosilicié à l'état de complexe évolue vers la formation de silanols sous forme aqueuse soluble présentant les propriétés des silanols de l'art antérieur.
Toutefois, quelque soit le dérivé silicié utilisé pour la préparation du complexe selon l'invention, il a été constaté que le silanol libéré après hydrolyse n'est pas obligatoirement le produit d'hydrolyse complet et direct du dérivé silanolé de départ.

Suivant un mode de réalisation avantageux de l'invention, la teneur en silicium dans les complexes selon l'invention varie entre 0,1 et 10 % en poids, de préférence entre 1 et 5 % en poids.

La présente invention est illustrée par les exemples qui suivent, qui n'ont bien entendu aucun caractère limitatif.

### Exemple 1 - Stabilisant lactose

100 ml de méthyltriéthoxysilane sont solubilisés dans un mélange de 80 g d'eau et de 350 g d'éthanol absolu. 1,1 Kg de lactose monohydrate est ensuite ajouté à la solution (partiellement soluble), et le tout est maintenu sous agitation à température ambiante pendant 17 heures. Les solvants sont ensuite progressivement éliminés par distillation sous pression réduite (2000-2600 Pa) en chauffant modérément. On obtient finalement 1,12 Kg d'un solide blanc pulvérulent, susceptible d'être par la suite transformé, par dissolution dans l'eau ou in vivo, pour former un silanol biologiquement actif ayant les propriétés des silanols de l'art antérieur.

### Exemple 2 - Stabilisant hydrolysat de collagène

100 g d'hydrolysat de collagène sont dissous à 30°C et sous agitation dans 900 g d'eau distillée, puis on ajoute 1 litre d'éthanol absolu. 100 g de méthyltriéthoxysilane sont alors ajoutés au mélange, au goutte à goutte et sous agitation. Le méthyltriéthoxysilane est insoluble dans l'eau mais il s'hydrolyse en méthylsilanetriol soluble qui vient se combiner aux polypeptides constitutifs de l'hydrolysat de collagène, et le mélange devient peu à peu limpide et homogène. La solution est maintenue sous agitation à température ambiante pendant 17 heures. On augmente ensuite la température jusqu'à 50°C avant de rajouter 300 g d'hydrolysat de collagène. A l'aide d'une solution d'acide chlorhydrique dilué, le milieu est maintenu à pH acide si nécessaire. L'éthanol et une partie de l'eau sont ensuite éliminés par distillation sous pression réduite (2000-2600 Pa) en chauffant modérément. On obtient un résidu plus ou moins gélifié selon la nature de l'hydrolysat de collagène et la quantité d'acide rajouté. On procède alors à une deshydratation poussée pour obtenir finalement un solide coloré, translucide qui peut être broyé pour former une poudre légérement colorée, parfaitement soluble dans l'eau.

### Exemple 3 - Stabilisant hydrolysat de collagène - Dispersant cellulose

100 g d'hydrolysat de collagène sont dissous à 30°C et sous agitation, dans 900 g d'eau distillée. On ajoute 1 litre d'éthanol absolu. 50 g de méthyltriéthoxysilane sont alors ajoutés au mélange, au goutte à goutte sous agitation. Il se produit une hydrolyse du méthyltriéthoxysilane en méthylsilanetriol, qui vient se combiner aux polypeptides constitutifs de l'hydrolysat de collagène, et le mélange devient rapidement limpide et homogène. La solution est maintenue sous agitation à température ambiante pendant 17 heures. On augmente ensuite la température jusqu'à 50°C avant de rajouter encore 100 g d'hydrolysat de collagène. A l'aide d'une solution d'acide chlorhydrique dilué, le milieu est acidifié si nécessaire puis on rajoute 200 g de cellulose microcristalline. On maintient sous agitation jusqu'à parfaite homogénéisation du mélange (la cellulose est insoluble). L'éthanol et une partie de l'eau sont ensuite éliminés par distillation sous pression réduite (2000-2600 Pa) en chauffant modérément. Après déshydratation sous vide poussé, on obtient un solide légèrement coloré, qui peut être broyé pour former une poudre parfaitement soluble dans l'eau.

### Exemple 4 - Stabilisant hydrolysat de protéines de blé

200 g d'un hydrolysat de protéines de blé sont dissous à température ambiante dans 500 g d'eau distillée. On ajoute ensuite 500 ml d'éthanol absolu. 25 g de méthyltriéthoxysilane sont alors ajoutés sur la solution, au goutte à goutte et sous agitation. L'hydrolyse du méthyltriéthoxysilane en méthylsilanetriol à pH acide s'accompagne d'une homogénéisation du mélange. On maintient la solution sous agitation à température ambiante pendant 17 heures. L'éthanol et une partie de l'eau sont ensuite éliminés par distillation sous pression réduite (2000-2600 Pa) en chauffant modérément. Les solvants résiduels sont enfin éliminés sous vide poussé (13,332 Pa). On obtient 230 g d'une poudre colorée, parfaitement soluble dans l'eau.

### Exemple 5 - Stabilisants : acide salicylique et hydrolysat de protéines de blé

100 g d'un hydrolysat de protéines de blé sont dissous à température ambiante dans 1 litre d'eau distillée. Le milieu est acidifié et on rajoute alors, progressivement et sous agitation, 1 litre d'éthanol absolu. 50 g de méthyltriéthoxysilane sont ensuite ajoutés au goutte à goutte sous agitation. La solution se trouble, puis assez rapidement redevient limpide et homogène. La solution est maintenue sous agitation à température ambiante pendant 17 heures. On rajoute ensuite 300 g de l'hydrolysat. Lorsque tout est dissous on réajuste éventuellement le pH avec de l'acide salicylique. L'éthanol et une partie de l'eau sont ensuite éliminés par distillation sous pression réduite (2000-2600 Pa) en chauffant modérément. Les solvants résiduels sont enfin éliminés sous vide poussé (13,332 Pa). On obtient 460 g d'une poudre colorée, parfaitement soluble dans l'eau.

## Revendications

1. Complexe à base d'un composé organosilicié de formule générale (I) suivante:
R₄ - Si (OR₁) (OR₂) (R₃) (I)
dans laquelle
R₁ et R₂ chacun indépendamment représentent un atome d'hydrogène ou un groupement alkyle,
R₃ représente un atome d'hydrogène, un groupement hydroxyle, un groupement amine,un groupement alkyle, un groupement alcoxy ou un groupement carboxylate,
et au moins un des groupements OR₁, OR₂ ou R₃ représente un groupement hydroxyle,
R₄ représente un groupement alkyle substitué ou non tel que notamment un groupement alkylphosphate ou alkylphosphonate, ou tout autre groupement tel que la liaison R₄-Si n'est pas hydrolysable, ledit complexe étant **caractérisé en ce que** :
- il est sous forme solide,
- il résulte de la formation de liaisons faibles ou fortes entre le composé organosilicié et au moins un stabilisant.

2. Complexe selon la revendication 1, dans lequel R₄ représente un groupement diéthylphosphatoéthyle et/ou les groupements OR₁, OR₂ et R₃ représentent chacun un groupement hydroxyle.

3. Complexe selon l'une quelconque des revendications 1 ou 2, **caractérisé en ce que** ledit stabilisant est un acide carboxylique, un acide aminé ou un dérivé d'acide aminé, un peptide ou une protéine, un alcool ou un polyol, un polysaccharide, et/ou leurs sels.

4. Complexe selon la revendication 3, **caractérisé en ce que** ledit acide carboxylique et/ou son sel est l'acide aspartique, l'acide glutamique, l'acide lactique, l'acide salicylique, l'acide théophylline acétique, l'acide pyrrolidinone carboxylique et/ou leurs sels.

5. Complexe selon la revendication 3, **caractérisé en ce que** ledit acide aminé et/ou son sel est l'arginine, la sérine, la thréonine, l'hydroxyproline, l'acétylméthionine, l'acétyl tyrosine et/ou leurs sels.

6. Complexe selon la revendication 3, **caractérisé en ce que** ledit peptide est un polypeptide dérivé de protéine d'élastine, de spiruline, de collagène, ou de protéine végétale, de préférence choisie parmi les protéines d'avoine ou de blé.

7. Complexe selon la revendication 6, **caractérisé en ce que** ledit polypeptide est un hydrolysat de protéine d'élastine, de spiruline, de collagène, ou de protéine végétale, de préference choisie parmi les protéines d'avoine ou de blé.

8. Complexe selon la revendication 3, **caractérisé en ce que** ladite protéine est une protéine d'élastine, de spiruline, de collagène, ou une protéine végétale, de préférence choisie parmi les protéines d'avoine ou de blé.

9. Complexe selon la revendication 3, **caractérisé en ce que** ledit polyol est le lactose.

10. Complexe selon la revendication 3, **caractérisé en ce que** ledit polysaccharide est un glycosaminoglycanne, un mucopolysaccharide, la pectine ou l'acide alginique.

11. Complexe selon la revendication 10, **caractérisé en ce que** le mucopolysaccharide est l'acide hyaluronique.

12. Complexe selon les revendications 1 à 11, **caractérisé en ce qu'**il est éventuellement dilué par au moins un dispersant.

13. Complexe selon l'une quelconque des revendications 12, **caractérisé en ce que** ledit dispersant est un polyamide, un carbohydrate, un polysaccharide, un polyoxyéthylène tel qu'un polyéthylène glycol ou un corps gras non soluble dans l'eau ou faiblement soluble dans l'eau.

14. Complexe selon la revendication 13, **caractérisé en ce que** ledit polysaccharide est de la cellulose.

15. Complexe selon la revendication 13, **caractérisé en ce que** ledit corps gras non soluble dans l'eau ou faiblement soluble dans l'eau est un triglycéride.

16. Composition pharmaceutique ou cosmétique **caractérisée en ce qu'**elle comprend un complexe selon l'une quelconque des revendications 1 à 15, en association avec tout excipient approprié.

## Patentansprüche

1. Komplex auf der Basis einer organischen Siliciumverbindung der folgenden allgemeinen Formel (I)
R₄- Si (OR₁) (OR₂) (R₃) (I)
in der
R₁ und R₂ jeweils unabhängig voneinander ein Wasserstoffatom oder eine Alkylgruppe darstellen,
R₃ ein Wasserstoffatom, eine Hydroxylgruppe, eine Aminogruppe, eine Alkylgruppe, eine Alcoxygruppe oder eine Carboxylatgruppe darstellt,
und mindestens eine der Gruppen OR₁, OR₂ oder R₃ eine Hydroxylgruppe darstellt,
R₄ eine substituierte oder nicht substituierte Alkylgruppe, wie z.B. insbesondere eine Alkylphosphat- oder Alkylphosphanatgruppe, oder jede andere Gruppe, die so beschaffen ist, dass die Bindung R₄-Si nicht hydrolysierbar ist, wobei dieser Komplex **dadurch gekennzeichnet ist, dass**:
- er in fester Form vorliegt,
- er sich aus der Bildung von schwachen oder starken Bindungen zwischen der organischen Siliciumverbindung und mindestens einem Stabilisator ergibt.

2. Komplex nach Anspruch 1, in dem R₄ eine Diethylphosphatoethylgruppe darstellt und/oder die Gruppen OR₁, OR₂ und OR₃ jeweils eine Hydroxylgruppe darstellen.

3. Komplex nach einem der Ansprüche 1 oder 2, **dadurch gekennzeichnet, dass** der Stabilisator eine Carbonsäure, eine Aminosäure oder ein Aminosäurederivat, ein Peptid oder ein Protein, ein Alkohol oder ein Polyol, ein Polysaccharid und/oder ihre Salze ist.

4. Komplex nach Anspruch 3, **dadurch gekennzeichnet, dass** die Carbonsäure und/oder ihr Salz Asparginsäure, Glutaminsäure, Milchsäure, Salicylsäure, Theophyllinessigsäure, Pyrrolidinoncarbonsäure und/oder ihre Salze sind.

5. Komplex nach Anspruch 3, **dadurch gekennzeichnet, dass** die Aminosäure und/oder ihr Salz Arginin, Serin, Threonin, Hydroxyprolin, Acetylmethionin, Acetyltyrosin und/oder ihre Salze sind.

6. Komplex nach Anspruch 3, **dadurch gekennzeichnet, dass** das Peptid ein Polypeptid ist, das von Elastin-, Spirolin-, Collagenprotein oder von pflanzlichem Protein, das vorzugsweise aus den Hafer- oder Weizenproteinen ausgewählt ist, abgeleitet ist.

7. Komplex nach Anspruch 6, **dadurch gekennzeichnet, dass** das Polypeptid ein Hydrolysat von Elastin-, Spirulin-, Collagenprotein oder von pflanzlichem Protein ist, das vorzugsweise aus den Hafer- oder Weizenproteinen ausgewählt ist.

8. Komplex nach Anspruch 3, **dadurch gekennzeichnet, dass** das Protein ein Elastin-, Spirulin-, Collagenprotein oder ein pflanzliches Protein ist, das vorzugsweise aus den Hafer- oder Weizenproteinen ausgewählt ist.

9. Komplex nach Anspruch 3, **dadurch gekennzeichnet, dass** das Polyol Lactose ist.

10. Komplex nach Anspruch 3, **dadurch gekennzeichnet, dass** das Polysaccharid ein Glykosaminoglykan, ein Mucopolysaccharid, Pectin oder Alginsäure ist.

11. Komplex nach Anspruch 10, **dadurch gekennzeichnet, dass** das Mucopolysaccharid Hyaluronsäure ist.

12. Komplex nach den Ansprüchen 1 bis 11, **dadurch gekennzeichnet, dass** er ggf. mit mindestens einem Dispersionsmittel verdünnt ist.

13. Komplex nach Anspruch 12, **dadurch gekennzeichnet, dass** das Dispersionsmittel ein Polyamid, ein Kohlehydrat, ein Polysaccharid, ein Polyoxyethylen, wie z.B. ein Polyethylenglykol, oder ein nicht wasserlösliches oder schwach wasserlösliches Fett ist.

14. Komplex nach Anspruch 13, **dadurch gekennzeichnet, dass** das Polysaccharid Cellulose ist.

15. Komplex nach Anspruch 13, **dadurch gekennzeichnet, dass** das nicht wasserlösliche oder schwach wasserlösliche Fett ein Triglycerid ist.

16. Pharmazeutische oder kosmetische Zusammensetzung, **dadurch gekennzeichnet, dass** sie einen Komplex nach einem der Ansprüche 1 bis 15 in Kombination mit jedem geeigneten Trägerstoff enthält.

## Claims

1. Complex based on an organo silicon compound having the following general formula (I) :
R₄- Si (OR₁) (OR₂) (R₃) (I)
wherein
R₁ and R₂ each one independently represent an atom of hydrogen or an alkyl group,
R₃ represents an hydrogen atom, an hydroxyl group, an amine group, an alkyl group, an alcoxy or a carboxylate group,
and at least one of the OR₁, OR₂ or R₃ groups represents an hydroxyl group,
R₄ represents an alkyl group substituted or not by a functional group such as, notably, an alkyl phosphate group or an alkyl phosphonate group, or any other group for which the R₄-Si bond is not hydrolyzable,
and the said complex is **characterized in that** :
- it is under solid form,
- it results of the formation of weak or strong bonds between the said organosilicon compound and at least one stabilizer.

2. Complex according to the claim 1 wherein R₄ represents a diethylphosphatoethyl group and/or OR₁, OR₂ and R₃ groups represent each an hydroxyl group.

3. Complex according to any of the claims 1 or 2, **characterized in that** the said stabilizer is a carboxylic acid, an amino acid or a derived amino acid, a peptide or a protein, an alcohol or a polyol, a polysaccharide, and/or their salts.

4. Complex according to the claim 3, **characterized in that** the said carboxylic acid and/or its salt is aspartic acid, glutamic acid, lactic acid, salicylic acid, theophylline acetic acid, carboxylic pyrrolidinone acid and/or their salts.

5. Complex according to the claim 3, **characterized in that** the said amino acid, and/or its salt is arginine, serine, threonine, hydroxyproline, acetylmethionine, acetyl tyrosine and/or their salts.

6. Complex according to the claim 3, **characterized in that** the said peptide is a polypeptide derived from elastine proteins, spirulina, collagen, or vegetal proteins, preferably chosen among oat or wheat proteins.

7. Complex according to the claim 6, **characterized in that** the said polypeptide is hydrolized elastin proteins, hydrolized spirulina, hydrolized collagen, or hydrolized vegetal proteins, preferably chosen among oat or wheat proteins.

8. Complex according to the claim 3, **characterized in that** the said protein is an elastin protein, spirulina protein, collagen protein, or a vegetal protein, preferably oat or wheat protein.

9. Complex according to the claim 3, **characterized in that** the said polyol is lactose.

10. Complex according to the claim 3, **characterized in that** the said polysaccharide is a glycosaminoglycanne, a mucopolysaccharide, pectin or alginic acid.

11. Complex according to the claim 10, **characterized in that** the said mucopolysaccharide is hyaluronic acid.

12. Complex according to one of the claims 1 to 11, **characterized in that** it is possibly diluted by at least one dispersant.

13. Complex according to whatever the claim 1 or 2, **characterized in that** the said dispersant is a polyamid, a carbohydrate, a polysaccharide, a polyoxyethylene such as a polyethylene glycol or a non water soluble (or weakly water soluble) fatty compound.

14. Complex according to the claim 13, **characterized in that** the said polysaccharide is cellulose.

15. Complex according to the claim 13, **characterized in that** the said non water soluble (or weakly water soluble) fatty compound is a triglyceride.

16. Cosmetic or pharmaceutical composition **characterized in that** the said compostion includes a complex according to whatever the aim 1 to 15, in association with any appropiated excipient.
